# EUROPÄISCHE PATENTSCHRIFT

(11) **EP 0 560 158 B1**
(45) Veröffentlichungstag und Bekanntmachung des Hinweises auf die Patenterteilung: **28.08.1996**
(21) Anmeldenummer: 93103213.0
(22) Anmeldetag: 01.03.1993
(51) Int. Cl.: C07C 227/08, C07C 227/10

(54) **Verfahren zur Herstellung von Beta-aminoacrylsäureestern**
Process for the preparation of beta-aminoacrylic acid esters
Procédé pour la préparation des esters de l'acide bêta-aminoacrylique

(30) Priorität: 12.03.1992 DE 4207852
(43) Veröffentlichungstag der Anmeldung: 15.09.1993
(73) Patentinhaber: BAYER AG, 51368 Leverkusen (DE)
(72) Erfinder: Kraus, Helmut, Dr., W-5000 Köln 80 (DE); Blank, Heinz-Ulrich, Dr., W-5068 Odenthal (DE); Marzolph, Gerhard, Dr., W-5000 Köln 80 (DE)

(56) Entgegenhaltungen:
- EP-A- 0 471 262
- CHEMISCHE BERICHTE, Bd. 104, 1971, Weinheim, DE, Seiten 2709 - 2727, H. BREDERECK et al: "Orthoamide, XVIII. Umsetzungen eines Aminalesters mit Carbonsäure- und Thiocarbonsäureamiden und -estern"
- TETRAHEDRON LETTERS, Bd. 27, Nr. 23, 1986, Oxford, GB, Seiten 2567 - 2570, P. F. SCHUDA et al: "The synthesis of Mannich bases from ketones and esters via enaminones"

## Beschreibung

Vorliegende Erfindung betrifft ein Verfahren zur Herstellung von β-Aminoacrylsäureestern aus Essigsäureestern und Aminalestern (Alkoxybis-(dialkylamino)-methanen).

Zur Herstellung von β-Aminoacrylsäureestern ist eine Reihe von Möglichkeiten bekannt. Ein interessanter technischer Weg ist die Umsetzung von Aminen mit β-Hydroxy-acrylsäureester-alkalisalzen, die aus CO, Alkoholat und Essigsäureestern in einer Druckreaktion, die 20 bis 50 bar erfordert, zugänglich sind. Dieser grundsätzlich aus Annales de Chimie 18 (1932), 108 bekannte Weg wurde in EP 217 018 auf etwa 70 % Gesamtausbeute verbessert. In EP 388 744 wird eine weitere Variante mit Ausbeuten von 85 bis 95 % der theoretischen Ausbeute beschrieben. In jedem Falle muß hierbei in Hochdruckapparaturen gearbeitet werden; die hohe Giftigkeit und die Brennbarkeit des CO erfordert besondere Sicherheitseinrichtungen.

Bei der Umsetzung von Acrylsäuremethylestern mit t-Butoxy-bis-(dimethylamino)-methan wird hochgiftiges Eisenpentacarbonyl verwendet (Tetrahedron Lett. 1976, 4061; An. Chem. 1980, 991). Bei diesem Verfahren muß zusätzlich mit der Polymerisation des Acrylesters gerechnet werden, was die Isolierung des Reaktionsproduktes erschwert.

Die Reaktion von t-Butoxy-bis-(dimethylamino)-methan mit Essigsäureethylester (Chem. Ber. 104 1971, 2709) ergibt nach 20-stündiger Reaktion bei 170°C 88 % β-Dimethylaminoacrylester. Allerdings muß man hierzu im Bombenrohr arbeiten und am Ende der Reaktion werden durch entstandenen Alkohol und Dimethylamin hohe Drücke erreicht. Bei weiterer Überprüfung dieser Reaktion wurde gefunden, daß bei Durchführung dieser Reaktion im 250 ml-, beziehungsweise 500 ml-Autoklaven nur noch 60 % der theoretischen Ausbeute erreicht werden (H. Saur, Dissertation, Univ. Stuttgart 1971, S. 41). Bei Verwendung von 50 % Überschuß an Essigester wird kein β-Dimethylaminoacrylester mehr gebildet; statt dessen erhält man eine höher siedende Substanz. Bei der Umsetzung von Methoxy-bis-(dimethylamino)-methan mit Buttersäureethylester wird überhaupt kein Umsetzungsprodukt erhalten (Org. Prep. Proceed. Int. 10, (1978), 67).

Die EP-A-471 262 betrifft ein Verfahren zur Herstellung substituierter 3-Aminoacrylester durch Umsetzung von Essigsäureestern mit Orthoameisensäurediamidestern bei Temperaturen von -35 bis 150°C unter vermindertem oder erhöhtem Druck, vorzugsweise unter Normaldruck. Die Verwendung tertiärer aromatischer Amine und tertiärer aromatischer Carbinole als Katalysatoren wird in der EP-A-471 262 weder erwähnt noch nahegelegt.

Es wurde ein Verfahren zur Herstellung von β-Aminoacrylsäureestern der Formel

(R⁴,R⁵)N-CH=CR¹-COOR² (I)

durch Umsetzung von Essigsäureestern der Formel

H₂CR¹-COOR² (II)

mit Aminalestern der Formel 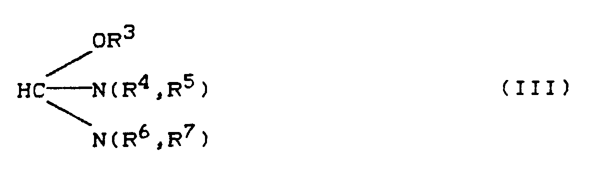
wobei in den Formeln
- R¹: Wasserstoff, geradkettiges oder verzweigtes C₁-C₈-Alkyl, geradkettiges oder verzweigtes C₂-C₈-Alkenyl, C₃-C₈-Cycloalkyl, C₆-C₁₂-Aryl, C₇-C₁₀-Aralkyl oder einen 5-8-gliedrigen aromatischen oder nichtaromatischen heterocyclischen Ring, dessen Heteroatome 1 oder 2 aus der Gruppe von N, O oder S sind, darstellt,
- R² und R³: unabhängig voneinander für geradkettiges oder verzweigtes C₁-C₈-Alkyl stehen und
- R⁴, R⁵, R⁶ und R⁷: unabhängig voneinander geradkettiges oder verzweigtes C₁-C₈-Alkyl, geradkettiges oder verzweigtes C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen aromatischen oder nichtaromatischen heterocyclischen Ring, dessen Heteroatome 1 oder 2 aus der Gruppe von N, O oder S sind, bedeuten, wobei weiterhin R⁴ und R⁵ beziehungsweise R⁶ und R⁷ gemeinsam mit dem N-Atom, das sie substituieren, einen 5 bis 8-gliedrigen aromatischen oder nichtaromatischen, N-heterocyclischen Ring bilden können, der ein weiteres Heteroatom aus der Gruppe N, O und S enthalten kann,
wobei die genannten Reste ihrerseits durch C₁-C₄-Alkyl, bevorzugt Methyl, durch C₁-C₄-Alkoxy, bevorzugt Methoxy, durch Halogen, wie Chlor, Fluor oder Brom, durch Phenyl oder Hydroxy oder, insbesondere für die aromatischen Teile der genannten Reste und Substituenten, durch typische aromatische Substituenten wie Nitro oder Cyano substituiert oder im Falle heterocyclischer Reste mit einem Benzolkern kondensiert sein können,
bei 0,5 bis 10 bar, bevorzugt 1 bis 5 bar, und bei 50 bis 170°C, bevorzugt bei 80 bis 150°C, in einem aprotischen polaren Lösungsmittel, bevorzugt aus der Gruppe der N-persubstituierten Säureamide, Sulfolane, Sulfoxide und Sulfone gefunden,
das dadurch gekennzeichnet ist, daß als Katalysatoren eine oder mehrere Verbindungen aus den Gruppen der tertiären aromatischen Amine und der tertiären aromatischen Carbinole der Formeln

Ar¹-N(R⁸,R⁹) (IV)

und 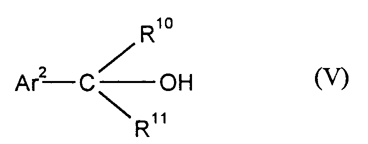 eingesetzt werden, wobei in den Formeln
- R⁸ und R⁹: unabhängig voneinander den oben genannten Bedeutungsumfang von R⁴ und R⁵ annehmen,
- Ar¹ und Ar²: unabhängig voneinander für 5- bis 7-gliedrige carbocyclische oder heterocyclische aromatische Reste stehen, die auch in der oben genannten Weise substituiert sein können,
- R¹⁰: den Bedeutungsumfang von R⁴ hat und
- R¹¹: den Bedeutungsumfang von R⁴ oder Ar² annimmt, von R⁴ und Ar² jedoch unabhängig ist.

Die Reaktion des erfindungsgemäßen Verfahrens kann in allgemeiner Form wie folgt dargestellt werden:

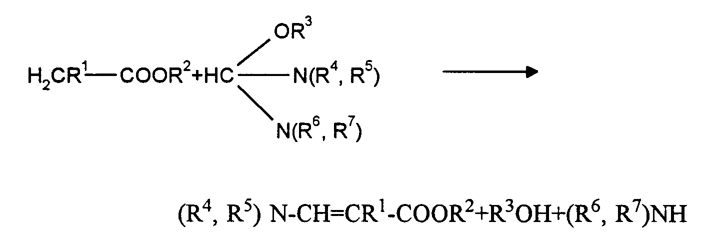

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle, Octyle, bevorzugt die genannten C₁-C₄-Alkylreste, besonders bevorzugt Methyl oder Ethyl, ganz besonders bevorzugt Methyl.

C₂-C₈-Alkenyl ist Vinyl, Propenyl, Allyl, die isomeren Butenyle, Amylenyle, Hexenyle oder Octenyle, bevorzugt die genannten C₃-C₄-Alkenylreste.

C₂-C₈-Alkoxyalkyl ist beispielsweise Methoxymethyl, Ethoxymethyl, Methoxyethyl sowie weitere Reste aus der Gruppe C₃-C₉-Alkyl, in welchem eine CH₂-Gruppe durch ein O-Atom ersetzt ist.

C₃-C₈-Alkoxyalkenyl ist beispielsweise Methoxyvinyl, Ethoxyvinyl, Methoxyallyl, 2-Methoxy-propenyl und andere Reste aus der Gruppe von C₄-C₉-Alkenyl, worin eine CH₂-Gruppe durch ein O-Atom ersetzt ist.

C₃-C₈-Cycloalkyl ist beispielsweie Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger aromatischer oder nichtaromatischer heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin.

Weiterhin können R⁴ und R⁵ beziehungsweise R⁶ und R⁷ gemeinsam mit dem N-Atom, das sie substituieren, einen 5- bis 8-gliedrigen aromatischen oder nichtaromatischen Ring bilden, der ein weiteres Heteroatom aus der Gruppe N, O und S enthalten kann, Solche Systeme sind beispielsweise Pyrrol, Pyrrolidin, Pyrrolin, Pyrazol, Pyrazolidin, Imidazol, Imidazolidin, Thiazol, Thiazolidin, Piperazin, Piperidin, Morpholin, Azepin, Dihydroazocin.

Für R¹ sei bevorzugt Wasserstoff oder geradkettiges oder verzweigtes C₁-C₄-Alkyl, besonders bevorzugt Wasserstoff und Methyl genannt. Für R² sei bevorzugt geradkettiges oder verzweigtes C₁-C₄-Alkyl, besonders bevorzugt Methyl oder Ethyl genannt. Für R³ sei bevorzugt geradkettiges oder verzweigtes C₁-C₅-Alkyl genannt.

In weiterhin bevorzugter Weise bedeuten R⁴, R⁵, R⁶ und R⁷ unabhängig voneinander geradkettiges oder verzweigtes C₁-C₄-Alkyl, Cyclopropyl, Cyclopentyl, Cyclohexyl, Phenyl oder Benzyl, wobei weiterhin R⁴ und R⁵ beziehungsweise R⁶ und R⁷ gemeinsam mit dem N-Atom, das sie substituieren, einen 5- bis 8-gliedrigen aromatischen oder nichtaromatischen N-heterocyclischen Ring bilden können, der ein weiteres Heteroatom aus der Gruppe N, O und S enthalten kann. Besonders bevorzugt stellen R⁴ bis R⁷ unabhängig voneinander geradkettiges oder verzweigtes C₁-C₄-Alkyl dar oder R⁴ und R⁵ beziehungsweise R⁶ und R⁷ bedeuten gemeinsam mit dem N-Atom, das sie substituieren, Morpholino, Pyrrolidino oder Piperidino.

Durch den niedrigen Druckbedarf des erfindungsgemäßen Verfahrens ist es erstmals möglich, diese interessante Reaktion unter günstigen technischen Umständen durchzuführen.

Aprotische polare Lösungsmittel für das erfindungsgemäße Verfahren stammen beispielsweise aus der Gruppe der N-persubstituierten Säureamide, wie Dimethylformamid (DMF), Dimethylacetamid (DMAC), Diethylacetamid und deren Homologe, N-Methyl-pyrrolidon (NMP), N-Methyl-caprolactam (NMC), Phosphorsäure-hexamethylamid, Tetramethylharnstoff und ähnliche; der Gruppe des Sulfolans und seiner durch Methyl, Ethyl und andere inerte Substituenten substituierten Derivate; der Gruppe der Sulfoxide, wie Dimethylsulfoxid, Diethylsulfoxid und andere; der Gruppe der Sulfone, wie Diethylsulfon, Dimethylsulfon und andere. Da auch die Einsatzstoffe und die Reaktionsprodukte des erfindungsgemäßen Verfahrens polaren und aprotischen Charakter haben, ist es möglich, einen Überschuß von ihnen als systemeigene Lösungs- und Verdünnungsmittel einzusetzen. Es ist sogar möglich, auf systemfremde aprotische polare Lösungsmittel völlig zu verzichten. Für den Fall, daß ein Fremdlösungsmittel eingesetzt wird, sei bevorzugt ein N-persubstituiertes Säureamid, besonders bevorzugt NMP, NMC, DMAC, DMF oder Tetramethyl-harnstoff, ganz besonders bevorzugt DMF, genannt.

Der Aminalester und der Essigester werden im Verhältnis 3:1 bis 1:10, bevorzugt 2:1 bis 1:5, besonders bevorzugt 1:1 bis 1:4, zur Reaktion gebracht.

Als Aminalester kann neben der reinen Verbindung auch das Dismutierungsgemisch aus Amidacetal, Aminalester und Tris-(Dialkylamino)-methan eingesetzt werden, welches im Verhältnis 0:1:0 bis 0,33:0,33:0,33 vorliegen kann. Herstellungsbedingt ist auch ein (höherer) Anteil an Amidacetal möglich und zulässig; er bewegt sich im Bereich von 0 bis 20 Gew.-% der Gesamtgewichtsmenge des Aminalesters, bzw. des reinen Dismutierungsgemisches. Diese Zusammenhänge sind dem Fachmann bekannt.

Die Reaktionsbedingungen der Aminomethylenierung von substituierten Essigsäureestern im Rahmen des erfindungsgemäßen Verfahrens können in einfacher Weise der Reaktivität der Reaktionspartner verschiedenen Substituenten angepaßt werden. So steigt die Reaktivität der Aminalester, wenn der Alkoxyrest-R³ von einem primären über einen sekundären zu einem tertiären Rest übergeht. Für tertiäre Aminalester ist bei Umsetzung im polaren aprotischen Lösungsmittel, bevorzugt in DMF, bereits Normaldruck ausreichend. Bei Aryl-substituierten Essigsäureestern ist kein zusätzliches Lösungsmittel nötig; es ist ausreichend, den jeweiligen Ester im Überschuß einzusetzen. Benutzt man primäre Aminalester, deren Herstellungskosten günstiger sind, wird man die Umsetzung im oberen Teil des genannten Temperaturbereiches durchführen; ein Druckanstieg auf über 10 bar, bevorzugt auf über 5 bar, ist jedoch auch hier nicht erforderlich. Die Alkoxygruppe des Aminalesters sollte mit der des Essigesters übereinstimmen, da sonst eine geringfügige Umesterung erfolgen kann.

Geeignete Reste Ar¹ und Ar² sind beispielsweise: Phenyl, Biphenyl, Nitrophenyl, Chlorphenyl, Tolyl, Xylyl, Pyridyl, Picolyl, Chlorpyridyl, Thiophenyl, Pyrryl.

Der Katalysator oder ein Gemisch mehrerer von ihnen wird in einer Menge von 0,1 bis 10 Mol-%, bevorzugt 0,5 bis 5 Mol-%, bezogen auf den Aminalester, eingesetzt. Hierbei werden im allgemeinen Aminalester und Essigester vorgelegt und der Katalysator nachgesetzt.

Der erfolgreiche Einsatz der genannten Katalysatoren ist überraschend, da bekannt ist, daß cyclische sekundäre Amine zwar die Reaktivität des DMF-dimethylacetals erhöhen, dabei jedoch ein Gemisch an Dialkylamino- und cyclischer Aminomethylenverbindung ergeben. Diese reaktionsbeschleunigende Wirkung cyclischer sekundärer Amine wurde auch bei Kondensationen mit Aminalestern bestätigt, wobei jedoch ebenfalls ein Gemisch der Aminoacrylester erhalten wird. Aliphatische tertiäre Amine, wie Triethylamine, die Umsetzungen mit dem DMF-acetal katalysieren sollen, bewiesen sich beim Aminalester als wirkungslos.

Bei der Aufarbeitung des Reaktionsgemisches fallen Katalysatoren im Destillationssumpf an. Die im Destillationssumpf verbliebenen Katalysatoren sind wieder verwendbar.

### Beispiel 1

In einem 300 ml-V₄A-Autoklaven wurden 34,6 g 96%iger Methylaminalester, 28 g Essigsäuremethylester und 1,5 g 4-Dimethylamino-pyridin in 80 ml DMF 8 h auf 130°C erhitzt. Der Maximaldruck betrug 5 bar, die Ausbeute lag bei 89,7 % der theoretischen Ausbeute.

### Beispiel 2

Der Versuch nach Beispiel 1 wurde mit Essigsäureethylester durchgeführt. Man erhielt 74,5 % β-Dimethylamino-acrylsäureethylester und 17,6 % des entsprechenden Methylesters.

### Beispiel 3

Zum Versuch nach Beispiel 1 wurde anstatt 4-Dimethylamino-pyridin 2,5 g 1,1-Diphenylmethanol zugesetzt. Man erhielt 88,2 % der theoretischen Ausbeute an β-Dimethylamino-acrylsäuremethylester.

### Beispiel 4 (zum Vergleich)

Der Versuch nach Beispiel 1 wurde ohne Katalysator durchgeführt. Man erhielt 82,0 % an Produkt.

## Patentansprüche

1. Verfahren zur Herstellung von β-Aminoacrylsäureestern der Formel
(R⁴,R⁵)N-CH=CR¹-COOR²
durch Umsetzung von Essigsäureestern der Formel
H₂CR¹-COOR²
mit Aminalestern der Formel 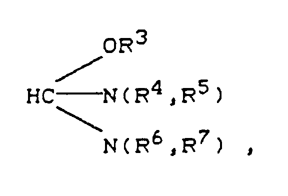 wobei in den Formeln
R¹ Wasserstoff, geradkettiges oder verzweigtes C₁-C₈-Alkyl, geradkettiges oder verzweigtes C₂-C₈-Alkenyl, C₃-C₈-Cycloalkyl, C₆-C₁₂-Aryl, C₇-C₁₀-Aralkyl oder einen 5- bis 8-gliedrigen aromatischen oder nichtaromatischen heterocyclischen Ring, dessen Heteroatome 1 oder 2 aus der Gruppe von N, O oder S sind, darstellt,
R² und R³ unabhängig voneinander für geradkettiges oder verzweigtes C₁-C₈-Alkyl stehen und
R⁴, R⁵, R⁶ und R⁷ unabhängig voneinander geradkettiges oder verzweigtes C₁-C₈-Alkyl, geradkettiges oder verzweigtes C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen aromatischen oder nichtaromatischen heterocyclischen Ring, dessen Heteroatome 1 oder 2 aus der Gruppe von N, O oder S sind, bedeuten, wobei weiterhin R⁴ und R⁵ beziehungsweise R⁶ und R⁷ gemeinsam mit dem N-Atom, das sie substituieren, einen 5 bis 8-gliedrigen aromatischen oder nichtaromatischen N-heterocyclischen Ring bilden können, der ein weiteres Heteroatom aus der Gruppe N, O und S enthalten kann,
wobei die genannten Reste ihrerseits durch C₁-C₄-Alkyl, durch C₁-C₄-Alkoxy, durch Halogen, durch Phenyl oder Hydroxy, durch Nitro oder Cyano substituiert oder im Falle heterocyclischer Reste mit einem Benzolkern kondensiert sein können,
bei 0,5 bis 10 bar und bei 50 bis 170°C in einem aprotischen polaren Lösungsmittel,
dadurch gekennzeichnet, daß ein oder mehrere Katalysatoren aus der Gruppe der aromatischen, tertiären Amine und der aromatischen Carbinole der Formeln
Ar¹-N(R⁸,R⁹) (IV)
und 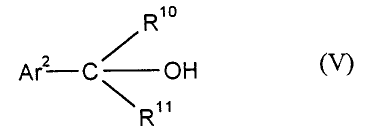 eingesetzt werden, wobei in den Formeln
R⁸ und R⁹ unabhängig voneinander den oben genannten Bedeutungsumfang von R⁴ und R⁵ annehmen,
Ar¹ und Ar² unabhängig voneinander für 5- bis 7-gliedrige carbocyclische oder heterocyclische aromatische Reste stehen, die auch substituiert sein können,
R¹⁰ den Bedeutungsumfang von R⁴ hat und
R¹¹ den Bedeutungsumfang von R⁴ oder Ar² annimmt, von R⁴ und Ar² jedoch unabhängig ist.

2. Verfahren nach Anspruch 1, dadurch gekennzeichnet, daß R¹ für Wasserstoff oder geradkettiges oder verzweigtes C₁-C₄-Alkyl, bevorzugt für Wasserstoff oder Methyl steht.

3. Verfahren nach Anspruch 1, dadurch gekennzeichnet, daß R² für geradkettiges oder verzweigtes C₁-C₄-Alkyl, bevorzugt für Methyl oder Ethyl steht.

4. Verfahren nach Anspruch 1, dadurch gekennzeichnet, daß R³ für geradkettiges oder verzweigtes C₁-C₅-Alkyl, bevorzugt für n- und iso-Alkyl, besonders bevorzugt für Methyl oder Ethyl steht.

5. Verfahren nach Anspruch 1, dadurch gekennzeichnet, daß R⁴, R⁵, R⁶ und R⁷ unabhängig voneinander geradkettiges oder verzweigtes C₁-C₄-Alkyl, Cyclopropyl, Cyclopentyl, Cyclohexyl, Phenyl oder Benzyl bedeuten und weiterhin R⁴ und R⁵ beziehungsweise R⁶ und R⁷ gemeinsam mit dem N-Atom, das sie substituieren, einen 5 bis 8-gliedrigen aromatischen oder nichtaromatischen N-heterocyclischen Ring bilden können, der ein weiteres Heteroatom aus der Gruppe N, O und S enthalten kann, bevorzugt unabhängig voneinander geradkettiges oder verzweigtes C₁-C₄-Alkyl, bedeuten und weiterhin R⁴ und R⁵ beziehungsweise R⁶ und R⁷ gemeinsam mit dem N-Atom, das sie substituieren, Morpholino, Pyrrolidino oder Piperidino bedeuten können.

6. Verfahren nach Anspruch 1, dadurch gekennzeichnet, daß man als aprotisches polares Lösungsmittel ein N-persubstituierter Säureamid, bevorzugt NMP, NMC, DMAC und DMF oder Tetramethylharnstoff, besonders bevorzugt DMF, verwendet.

7. Verfahren nach Anspruch 1, dadurch gekennzeichnet, daß man Aminalester und Essigester im Verhältnis 3:1 bis 1:10, bevorzugt 2:1 bis 1:5, besonders bevorzugt 1:1 bis 1:4 einsetzt.

8. Verfahren nach Anspruch 1, dadurch gekennzeichnet, daß man statt des einen Aminalesters ein Dismutierungsgemisch, das herstellungsbedingt auch einen (höheren) Anteil an Amidacetal enthalten kann, einsetzt.

## Claims

1. Process for the preparation of β-aminoacrylic acid esters of the formula
(R⁴,R⁵)N-CH=CR¹-COOR²
by reacting acetic acid esters of the formula
H₂CR¹-COOR²
with aminal esters of the formula 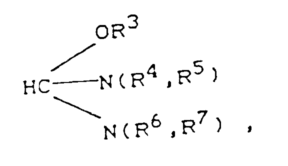 in which formulae
R¹ is hydrogen, linear or branched C₁-C₈-alkyl, linear or branched C₂-C₈-alkenyl, C₃-C₈-cycloalkyl, C₆-C₁₂-aryl, C₇-C₁₀-aralkyl or a 5- to 8-membered aromatic or non-aromatic heterocyclic ring in which the heteroatoms are 1 or 2 from the group consisting of N, O or S,
R² and R³ independently of one another are linear or branched C₁-C₈-alkyl and
R⁴, R⁵, R⁶ and R⁷ independently of one another are linear or branched C₁-C₈-alkyl, linear or branched C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl or a 5- to 8-membered aromatic or non-aromatic heterocyclic ring in which the heteroatoms are 1 or 2 from the group consisting of N, O or S, it further being possible for R⁴ and R⁵ or R⁶ and R⁷, together with the N atom which they substitute, to form a 5- to 8-membered aromatic or non-aromatic N-heterocyclic ring which can contain another heteroatom from the group consisting of N, O and S,
it being possible for the said radicals in turn to be substituted by C₁-C₄-alkyl, by C₁-C₄-alkoxy, by halogen, by phenyl or hydroxyl, or by nitro or cyano, or, in the case of heterocyclic radicals, to be fused with a benzene ring,
at 0.5 to 10 bar and at 50 to 170°C in an aprotic polar solvent, characterised in that one or more catalysts from the group consisting of aromatic tertiary amines and aromatic carbinols of the formulae
Ar¹-N(R⁸,R⁹) (IV)
and 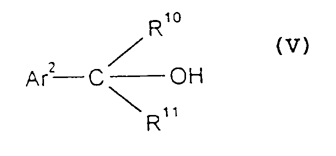 are used, in which formulae
R⁸ and R⁹ independently of one another have the range of meanings of R⁴ and R⁵ mentioned above,
Ar¹ and Ar² independently of one another are 5-to 7-membered carbocyclic or heterocyclic aromatic radicals which can also be substituted,
R¹⁰ has the range of meanings of R⁴ and
R¹¹ has the range of meanings of R⁴ or Ar², but is independent of R⁴ and Ar².

2. Process according to Claim 1, characterised in that R¹ is hydrogen or linear or branched C₁-C₄-alkyl, preferably hydrogen or methyl.

3. Process according to Claim 1, characterised in that R² is linear or branched C₁-C₄-alkyl, preferably methyl or ethyl.

4. Process according to Claim 1, characterised in that R³ is linear or branched C₁-C₅-alkyl, preferably n-and iso-alkyl and particularly preferably methyl or ethyl.

5. Process according to Claim 1, characterised in that R⁴, R⁵, R⁶ and R⁷ independently of one another are linear or branched C₁-C₄-alkyl, cyclopropyl, cyclopentyl, cyclohexyl, phenyl or benzyl, it further being possible for R⁴ and R⁵ or R⁶ and R⁷, together with the N atom which they substitute, to form a 5-to 8-membered aromatic or non-aromatic N-heterocyclic ring which can contain another heteroatom from the group consisting of N, O and S, and are preferably independently of one another linear or branched C₁-C₄-alkyl, it further being possible for R⁴ and R⁵ or R⁶ and R⁷, together with the N atom which they substitute, to be morpholino, pyrrolidino or piperidino.

6. Process according to Claim 1, characterised in that the aprotic polar solvent used is an N-persubstituted acid amide, preferably NMP, NMC, DMAC and DMF or tetramethylurea, and particularly preferably DMF.

7. Process according to Claim 1, characterised in that aminal ester and acetic acid ester are used in a ratio of 3:1 to 1:10, preferably 2:1 to 1:5 and particularly preferably 1:1 to 1:4.

8. Process according to Claim 1, characterised in that the one aminal ester is replaced with a dismutation mixture which, depending on the preparation, can also contain a (relatively high) proportion of amide acetal.

## Revendications

1. Procédé pour la préparation d'esters β-aminoacryliques de formule
(R⁴,R⁵)N-CH=CR¹-COOR² (I)
par réaction d'esters acétiques de formule
H₂CR¹-COOR² (II)
avec des esters d'aminals de formule 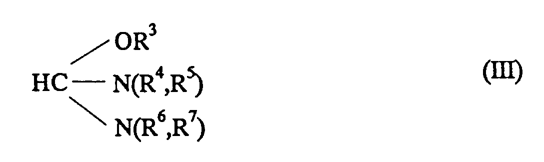 les symboles de ces formules ayant les significations suivantes
R¹ représente l'hydrogène, un groupe alkyle à chaîne droite ou ramifiée en C₁-C₈, un groupe alcényle à chaîne droite ou ramifiée en C₂-C₈, un groupe cycloalkyle en C₃-C₈, aryle en C₆-C₁₂, aralkyle en C₇-C₁₀ ou un noyau hétérocyclique aromatique ou non aromatique de 5 à 8 chaînons qui peut contenir 1 ou 2 hétéroatomes choisis parmi N, O ou S,
R² et R³ représentent chacun, indépendamment l'un de l'autre, un groupe alkyle à chaîne droite ou ramifiée en C₁-C₈ et
R⁴, R⁵, R⁶ et R⁷ représentent chacun, indépendamment les uns des autres, un groupe alkyle à chaîne droite ou ramifiée en C₁-C₈, un groupe alcényle à chaîne droite ou ramifiée en C₂-C₈, un groupe alcoxyalkyle en C₂-C₈, alcoxyalcényle en C₃-C₈, cycloalkyle en C₃-C₈, aryle en C₆-C₁₂, aralkyle en C₇-C₁₀ ou un noyau hétérocyclique aromatique ou non aromatique de 5 à 8 chaînons contenant 1 ou 2 hétéroatomes choisis parmi N, O et S, R⁴ et R⁵ d'une part, R⁶ et R⁷, d'autre part, pouvant également former ensemble et avec l'atome d'azote dont ils sont substituants un noyau N-hétérocyclique aromatique ou non aromatique de 5 à 8 chaînons qui peut contenir un autre hétéroatome choisi parmi N, O et S, les groupes mentionnés pouvant eux-mêmes être substitués par des groupes alkyles en C₁-C₄, alcoxy en C₁-C₄, des halogènes, des groupes phényle ou hydroxy, nitro ou cyano, et dans le cas de groupes hétérocycliques, il peut y avoir condensation avec un noyau benzénique,
à des pressions de 0,5 à 10 bar et des températures de 50 à 170°C, dans un solvant polaire aprotonique
caractérisé en ce l'on utilise un ou plusieurs catalyseurs pris dans le groupe des amines aromatiques tertiaires et des carbinols aromatiques de formules
Ar¹-N(R⁸,R⁹) (IV)
et 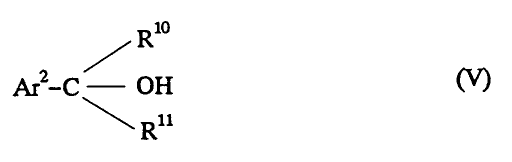 dans lesquelles
R⁸ et R⁹ ont chacun, indépendamment l'un de l'autre, l'une des significations indiquées pour R⁴ et R⁵,
Ar¹ et Ar² représentent chacun, indépendamment l'un de l'autre, un groupe aromatique carbocyclique ou hétérocyclique de 5 à 7 chaînons qui peut également être substitué,
R¹⁰ a l'une des significations de R⁴ et
R¹¹ a l'une des significations de R⁴ ou de Ar² mais indépendamment de ceux-ci.

2. Procédé selon la revendication 1, caractérisé en ce que R¹ représente l'hydrogène ou un groupe alkyle à chaîne droite ou ramifiée en C₁-C₄, de préférence l'hydrogène ou un groupe méthyle.

3. Procédé selon la revendication 1, caractérisé en ce que R² représente un groupe alkyle à chaîne droite ou ramifiée en C₁-C₄, de préférence un groupe méthyle ou éthyle.

4. Procédé selon la revendication 1, caractérisé en ce que R³ représente un groupe alkyle à chaîne droite ou ramifiée en C₁-C₅, de préférence un groupe n- ou iso-alkyle et dans les meilleures conditions un groupe méthyle ou éthyle.

5. Procédé selon la revendication 1, caractérisé en ce que R⁴, R⁵, R⁶ et R⁷ représentent chacun, indépendamment les uns des autres, un groupe alkyle à chaîne droite ou ramifiée en C₁-C₄, cyclopropyle, cyclopentyle, cyclohexyle, phényle ou benzyle, R⁴ et R⁵ d'une part, R⁶ et R⁷ d'autre part, pouvant également former avec l'atome d'azote dont ils sont substituants un noyau N-hétérocyclique aromatique ou non aromatique de 5 à 8 chaînons qui peut contenir un autre hétéroatome choisi parmi N, O et S, et de préférence R⁴, R⁵, R⁶ et R⁷ représentent chacun, indépendamment les uns des autres, un groupe alkyle à chaîne droite ou ramifiée en C₁-C₄, R⁴ et R⁵ d'une part, R⁶ et R⁷ d'autre part, pouvant également représenter avec l'atome d'azote dont ils sont substituants un cycle morpholino, pyrrolidino ou pipéridino.

6. Procédé selon la revendication 1, caractérisé en ce que l'on utilise en tant que solvant polaire aprotonique un amide entièrement substitué à l'azote, de préférence la N-méthylpyrrolidone, le N-méthylcaprolactame, le diméthylacétamide, le diméthylformamide ou la tétraméthylurée, plus spécialement le DMF.

7. Procédé selon la revendication 1, caractérisé en ce que l'on met en oeuvre l'ester d'aminal et l'ester acétique dans des proportions relatives de 3:1 à 1:10, de préférence de 2:1 à 1:5 et plus spécialement de 1:1 à 1:4.

8. Procédé selon la revendication 1, caractérisé en ce que l'on utilise à la place d'un ester d'aminai un mélange de dismutation qui, en raison des conditions de la préparation, peut également contenir une certaine proportion (forte) d'amidacétal.
